(19) Europäisches Patentamt
European Patent Office
Office européen des brevets

(11) EP 0 829 260 B1

(12) FASCICULE DE BREVET EUROPEEN

(45) Date de publication et mention de la délivrance du brevet:
**15.05.2002 Bulletin 2002/20**

(51) Int Cl.[7]: **A61K 7/48**, A61K 7/06, A61K 7/42

(21) Numéro de dépôt: **97401967.1**

(22) Date de dépôt: **21.08.1997**

# (54) Utilisation d'au moins un dérivé de pyrimidine 3-oxyde, substitué en 6 en tant que stimulateur de tyrosinase

Verwendung mindestens eines in 6-Stellung substituierten Pyrimidin-3-oxids zur Stimulierung von Tyrosinase

Use of at least one pyrimidine derivative substituted in position 6 as stimulant of tyrosinase

(84) Etats contractants désignés:
**AT BE CH DE DK ES FI FR GB GR IE IT LI LU MC NL PT SE**

(30) Priorité: **17.09.1996 FR 9611316**

(43) Date de publication de la demande:
**18.03.1998 Bulletin 1998/12**

(73) Titulaire: **L'OREAL**
**75008 Paris (FR)**

(72) Inventeurs:
- **Pruche, Francis**
**75018 Paris (FR)**
- **Gerst, Catherine**
**92600 Asnieres (FR)**

(74) Mandataire: **Tezier Herman, Béatrice**
**L'OREAL-DPI**
**6 rue Bertrand Sincholle**
**92585 Clichy Cédex (FR)**

(56) Documents cités:
**EP-A- 0 736 300**

- **STN, Serveur de Bases de Données, XP002029091**
- **STN, Serveur de Bases de Données, XP002029092**
- **STN, Serveur de Bases de Données, XP002029093**
- **DATABASE WPI Week 8647 Derwent Publications Ltd., London, GB; AN 86-308507 XP002029095 & JP 61 227 518 A (KANEBO)**
- **STN, Serveur de Bases de Données, XP002029094 & RUSHTON ET AL.: "quantitative assessment of 2% topical minoxidil in the treatment of male pattern baldness" CLIN. EXP. DERMATOL., vol. 14, no. 1, 1989, GB, pages 40-46,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 829 260 B1

Printed by Jouve, 75001 PARIS (FR)

## Description

L'invention concerne l'utilisation dans une composition pour favoriser la pigmentation de la peau et/ou des cheveux, d'au moins un dérivé de pyrimidine 3-oxyde, substitué en 6, ainsi qu'une composition comprenant au moins un de ces dérivés et son utilisation.

La couleur des cheveux et de la peau humaine est fonction de différents facteurs et notamment des saisons de l'année, de la race, du sexe et de l'âge. Elle est principalement déterminée par la concentration de mélanine produite par les mélanocytes. Les mélanocytes sont les cellules spécialisées qui par l'intermédiaire d'organelles particuliers, les mélanosomes, synthétisent la mélanine.

La synthèse de la mélanine ou mélanogénèse est particulièrement complexe et fait intervenir schématiquement les principales étapes suivantes :

$$\text{Tyrosine} \rightarrow \text{Dopa} \rightarrow \text{Dopaquinone} \rightarrow \text{Dopachrome} \rightarrow \text{Mélanine}$$

La tyrosinase (monophénol dihydroxyl phénylalanine : oxygen oxydoreductase EC 1,14,18,1) est l'enzyme essentielle intervenant dans cette suite de réactions. Elle catalyse notamment la réaction de transformation de la tyrosine en Dopa (dihydroxyphénylalanine) et la réaction de transformation de la Dopa en Dopaquinone.

Bien que le taux de mélanine varie d'une population à une autre, la quantité de tyrosinase ne varie pas significativement et le taux d'ARN messagers de la tyrosinase est identique dans des peaux blanches ou noires. Les variations dans la mélanogénèse sont donc dues à des variations de l'activité de la tyrosinase.

On sait que dans la plupart des populations la coloration brune de la peau et le maintien d'une coloration constante de la chevelure sont des aspirations importantes.

Il existe par ailleurs des maladies de la pigmentation comme par exemple le vitiligo qui est une maladie auto-immune qui se caractérise par l'apparition de plaques blanches sur la peau liées à un défaut de pigmentation.

Il existe donc un réel besoin de produit facilitant et/ou améliorant la pigmentation de la peau et/ou des cheveux.

A cet égard il a été proposé de nombreuses solutions dans le domaine de la coloration artificielle, par apport de colorants exogènes sensés donner à la peau et/ou aux cheveux une coloration le plus proche possible de ce qu'elle est naturellement ou dans le domaine de la coloration naturelle par stimulation des voie naturelle de pigmentation.

D'excellents résultats sont certes obtenus par les solutions proposées dans l'art antérieur, mais il n'en demeure pas moins que la stimulation de la pigmentation de la peau et/ou des cheveux par la voie naturelle reste la voie idéale de pigmentation.

A cet égard il a été proposé dans les documents WO-A-9517161, WO-9511003, WO-A-9501773, WO-A-9404674, WO-A-9404122, EP-A-585018, WO-A-9310804, WO-A-9220322 ou WO-A-9107945 des solutions aussi variées que des compositions contenant un inhibiteurs de phosphodiestérases, l'utilisation de prostaglandine, de fragments d'ADN, de dérivés de la tyrosine ou encore d'extraits de plantes.
Souvent les composés utilisés présentent des effets secondaires non négligeables ou sont des mélanges complexes qui ne présentent pas de spécificité.

Il a d'autre part été suggéré par Rushton et collaborateurs (Rushton, D.H., et coll., Clin. Exp. Dermatol., 1989, 14(1), 40-46) que le Minoxidil ou 2,4-diamino-6-piperidino pyrimidine 3-oxyde, pouvait avoir un effet stimulateur de la pigmentation du velus chez des hommes chauves traités par ce composé.

Le "Minoxidil" est connu pour ses effets anti-hypertenseur et pour sa capacité à favoriser la pousse des cheveux. Ces propriétés sont décrites dans US 4 596 812.

Le Minoxidil, s'il reste le composé de référence dans le domaine de la pousse des cheveux, présente des effets secondaires non négligeables qui rendent son utilisation délicate.
La découverte de substances ayant un effet sur la pigmentation de la peau et/ou des cheveux sans présenter d'effets secondaires gênants reste un objectif majeur de la recherche.

Un des buts de la présente invention est donc de proposer l'utilisation de nouveaux composés pour favoriser la pigmentation de la peau et/ou des cheveux tout en limitant les effets secondaires néfastes.

La demanderesse a maintenant découvert que certains dérivés de pyrimidine 3-oxyde, substitués en 6 ont un effet activateur de la tyrosinase similaire ou supérieur à celui du Minoxidil.

Les dérivés utilisables dans l'invention répondent à la formule générale (I) :

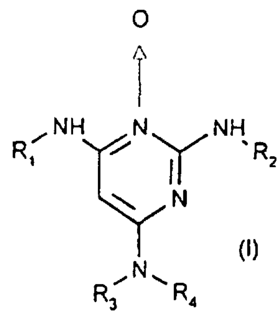

(I)

dans laquelle

$R_1$ et $R_2$. identiques ou différents, représentent un atome d'hydrogène ou un radical alkyle en $C_1$ - $C_{12}$;
$R_3$ et $R_4$ identiques ou différents, représentent un radical alkyle en $C_1$ - $C_{12}$, ou pris ensemble peuvent former avec l'atome d'azote auquel ils sont reliés un hétérocycle ;

étant entendu que lorsque R3 et R4 pris ensemble forment un cycle pipéridino alors au moins l'un des radicaux R1 ou R2 doit être différent d'un atome d'hydrogène.

Ainsi, l'invention a pour objet l'utilisation dans une composition cosmétique ou pour la préparation d'une composition pharmaceutique, à titre de principe actif, d'une quantité suffisante d'au moins un dérivé de pyrimidine 3-oxyde, substitué en 6 répondant à la formule générale I, ce dérivé ou la composition étant destiné à favoriser la pigmentation de la peau et/ou des cheveux.

L'invention a également pour objet l'utilisation dans une composition cosmétique ou pour la préparation d'une composition pharmaceutique, à titre de principe actif, d'une quantité suffisante d'au moins un dérivé de pyrimidine 3-oxyde, substitué en 6 répondant à la formule générale I, ce dérivé ou la composition étant destiné à stimuler l'activité tyrosinase.

L'invention concerne également les isomères optiques, seuls ou en mélange en toutes proportions, les formes acylées ou encore les sels pharmaceutiquement acceptables de ces dérivés.

Par radical alkyle en $C_1$ - $C_{12}$ on entend de préférence selon l'invention les radicaux alkyles linéaires ou ramifiés en $C_1$ - $C_{12}$ , éventuellement substitués par au moins un radical hydroxyle ou un radical benzyle et en particulier les formes linéaires ou ramifiées éventuellement substituées des radicaux méthyle, éthyle, propyle, butyle, pentyle, hexyle ou encore benzyle.

Par hétérocycle on entend tout cycle saturé ou insaturé, comportant au moins un atome d'azote dont en particulier ceux du groupe constitué par les cycles aziridino, azétidino, pyrrolidino, pipéridino, héxaméthylènimino, heptaméthylènimino, octaméthylènimino, tétrahydropyridino, dihydropyridino, pyrrole, pyrrazole, imidazole, triazole, alkyl-4 pipérazino, morpholino, thiomorpholino.
De préférence selon l'invention, l'hétérocycle est un cycle pipéridino.

Bien entendu selon l'invention il est possible d'utiliser les dérivés de formule (I) seuls ou en mélange en toute proportion.

Par tyrosinase, il faut entendre dans la présente invention toute enzyme présentant une activité tyrosinase, cette enzyme pouvant présenter d'autres activités enzymatiques.
L'activité tyrosinase peut se définir comme l'activité enzymatique qui catalyse l'oxydation de la tyrosine pour conduire à la formation du précurseur de mélanine : la Dopaquinone.

Il est connu de nombreux dérivés de pyrimidine substitués en 6. Parmi ceux-ci on peut notamment citer ceux décrits dans les demandes de brevet EP-A-353123, EP-A-356271, EP-A-408442, EP-A-420707, EP-A-427625, EP-A-459890, EP-A-519819, EP-A-522964, EP-A-525964, EP-A-540629.

Parmi les dérivés de pyrimidine 3-oxyde, substitués en 6 utilisables selon l'invention, on peut plus particulièrement citer :

le 2,4 bis-méthylamino-6-diméthylamino pyrimidine 3-oxyde,
le 2,4 bis-éthylamino-6-diméthylamino pyrimidine 3-oxyde,
le 2,4 bis-propylamino-6-diméthylamino pyrimidine 3-oxyde,
le 2,4 bis-propylamino-6-pipéridino pyrimidine 3-oxyde,
le 2 amino-4-méthylamino-6-pipéridino pyrimidine 3-oxyde,
le 2-amino-4-propylamino-6-pipéridino pyrimidine 3-oxyde,
le 2 amino-4-hexylamino-6-pipéridino pyrimidine 3-oxyde,

le 2 amino-4-benzylamino-6-pipéridino pyrimidine 3-oxyde,
le 2 amino-4-(2-hydroxyéthylamino)-6-pipéridino pyrimidine 3-oxyde,
le 2-amino-4-propylamino-6-diméthylamino pyrimidine 3-oxyde,
le 2-amino-4-butylamino-6-diméthylamino pyrimidine 3-oxyde,
le 2-amino-4-isopropylamino-6-diméthylamino pyrimidine 3-oxyde.

Et plus préférentiellement encore, on utilise selon l'invention :

le 2-amino-4-propylamino-6-diméthylamino pyrimidine 3-oxyde,
le 2 amino-4-méthylamino-6-pipéridino pyrimidine 3-oxyde,
le 2,4 bis-propylamino-6-diméthylamino pyrimidine 3-oxyde,
le 2,4 bis-propylamino-6-pipéridino pyrimidine 3-oxyde,
le 2,4 bis-méthylamino-6-diméthylamino pyrimidine 3-oxyde,
le 2,4 bis-éthylamino-6-diméthylamino pyrimidine 3-oxyde.

La quantité de dérivé nécessaire pour favoriser la pigmentation de la peau et/ou des cheveux est une quantité nécessaire pour stimuler l'activité tyrosinase. Elle est bien entendu dépendante de la nature du dérivé et de la nature de la tyrosinase en question et peut varier dans une large mesure.
Pour donner un ordre de grandeur, si on utilise le dérivé selon l'invention dans une composition cosmétique, la quantité de dérivé utilisable peut être comprise entre 0,01% et 20% du poids total de la composition et préférentiellement comprise entre 0,1% et 10% du poids total de la composition.
Pour donner un ordre de grandeur, si on utilise le dérivé selon l'invention dans une composition pharmaceutique, la quantité de dérivé utilisable peut être comprise entre 1% et 30% du poids total de la composition et préférentiellement comprise entre 2% et 15% du poids total de la composition.
Les compositions selon l'invention sont essentiellement destinées à favoriser la pigmentation de la peau et/ou des cheveux et à stimuler l'activité tyrosinase endogène de la peau et/ou des cheveux.
Un autre objet de l'invention concerne donc une composition cosmétique ou pharmaceutique comprenant au moins un dérivé répondant à la formule générale (I) et au moins un substrat d'au moins une enzyme présentant une activité tyrosinase.
Parmi les substrats utilisables selon l'invention on peut citer par exemple la tyrosine ou ses dérivés ou la dihydroxy-3,4 phényl $\alpha$-alanine (DOPA).
Il est possible de réunir en une seule composition le dérivé et le substrat. Mais des formes particulières de réalisation peuvent être envisagées, en particulier le dérivé et le substrat peuvent être administrés de manière simultanée, séparée ou étalée dans le temps.
Ainsi, l'invention a pour objet un produit comprenant au moins un dérivé répondant à la formule générale (I) et au moins un substrat d'au moins une enzyme présentant une activité tyrosinase comme produit de combinaison pour une utilisation simultanée, séparée ou étalée dans le temps pour favoriser la pigmentation de la peau et/ou des cheveux.
Sous une forme de réalisation particulière le dérivé et le substrat peuvent être conditionnés de manière séparée sous forme d'un kit dont les composants seront mélangés extemporanément.
L'invention a ainsi pour objet un kit conprenant au moins un dérivé répondant à la formule générale (I) et au moins un substrat d'au moins une enzyme présentant une activité tyrosinase pour une utilisation simultanée, séparée ou étalée dans le temps pour favoriser la pigmentation de la peau et/ou des cheveux.
La composition selon l'invention peut être ingérée, injectée ou appliquée sur la peau (sur toute zone cutanée du corps) ou les cheveux. Selon le mode d'administration, la composition selon l'invention peut se présenter sous toutes les formes galéniques normalement utilisées.
Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.
Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose$^R$ 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins composé de formule (I) à d'autres agents actifs. Parmi ces agents actifs, on peut citer à titre d'exemple :

- les agents améliorant l'activité sur la repousse et/ou sur le freinage de la chute des cheveux, et ayant déjà été décrits pour cette activité comme par exemple les esters d'acide nicotinique, dont notamment le nicotinate de tocophérol, le nicotinate de benzyle et les nicotinates d'alkyles en $C_1$-$C_6$ comme les nicotinates de méthyle ou d'hexyle, les agents favorisant la repousse des cheveux comme ceux décrits par la demanderesse dans la demande de brevet européen publiée sous le numéro 0648488 ;
- les agents diminuant la différenciation et/ou la prolifération tels que l'acide rétinoïque et ses isomères, le rétinol et ses esters, la vitamine D et ses dérivés, les oestrogènes tels que l'oestradiol ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés

de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde, dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale ou bactérienne.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétrayénoïque et eicosatriyénoïque ou leurs esters et amides, la vitamine D et ses dérivés, des extraits d'origine végétale ou bactérienne.

Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

On peut également envisager que la composition comprenant au moins un dérivé tel que défini précédement soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

La composition pharmaceutique selon l'invention peut être administrée par voie parentérale, entérale ou encore par voie topique. De préférence, la composition pharmaceutique est administrée par voie topique.

Afin de déterminer l'activité des dérivés de pyrimidine substitué en 6, la demanderesse a utilisé un procédé simple et rapide de mesure consistant à incuber dans un milieu adéquat une tyrosinase éventuellement purifiée et un de ses substrats, en présence d'un composé à tester, et à comparer les mesures réalisées avec les résultats de mesures identiques effectuées lors de l'incubation de la tyrosinase, éventuellement purifiée, avec un de ses substrats en l'absence du composé à tester.

On va maintenant donner à titre d'illustration des exemples qui ne sauraient limiter en aucune façon la portée de l'invention.

<u>Exemple 1</u> : Modulation de l'activité de la tyrosinase par les dérivés de pyrimidine 3-oxyde, substitué en 6 :

<u>Principes généraux de mesure</u> :

Une tyrosinase purifiée est incubée en présence de l'un de ses substrats dans un milieu approprié, en présence ou en l'absence d'un dérivé de pyrimidine 3-oxyde, substitué en 6. L'activité de l'enzyme est évaluée par mesure de la quantité d'un produit de transformation du substrat formé au cours de la réaction. La comparaison du résultat obtenu en présence d'un dérivé est comparé au résultat obtenu en l'absence de dérivé.
Cette comparaison permet d'évaluer l'influence du dérivé sur l'activité de l'enzyme.

<u>Préparation aux mesures</u> :

Le substrat utilisé est de la L-Tyrosine (vendu par la société SIGMA) dont il est préparé une solution mère dans du tampon phosphate salin (PBS) à la concentration de 0,5 mM.
L'enzyme utilisé est une tyrosinase de champignon (EC 1.14.18.1) purifiée vendue par la société SIGMA. Une solution

mère de cette enzyme est préparée dans du PBS à la concentration de 5 mg/ml ce qui représente une concentration d'environ 19500 unités internationales de tyrosinase par millilitre.
Le produit de transformation de la tyrosine par la tyrosinase, le Dopachrome, formé est mesuré par spectrophotométrie à la longueur d'onde de 475 nm à l'aide d'un appareil de type Perkin Elmer
Le dérivé à tester est préparé en solution dans du PBS à 1% d'éthanol à la concentration de 1 mM.

Mesures :

Dans une cuve à spectrophotomètre on mélange

500 µl de solution de tyrosine
390 µl de tampon phosphate
10 µl de solution de tyrosinase de champignon
100 µl de solution de dérivé à tester ou de PBS/éthanol 1% (témoin).

Le mélange est alors incubé à la température de 37°C et le Dopachrome formé est mesuré en continu pendant au moins 30 minutes.

Résultats :

Ces résultats sont exprimés en % d'activation de l'activité tyrosinase par rapport à la valeur obtenue avec le témoin (en l'absence de dérivé).

| Dérivés | Activation |
|---|---|
| Témoin | 0 % |
| 2,4 diamino-6-piperidino pyrimidine 3-oxyde, (Minoxidil) | 41 % |
| 2,4 bis-méthylamino-6-diméthylamino pyrimidine 3-oxyde, | 61 % |
| 2,4 bis-éthylamino-6-diméthylamino pyrimidine 3-oxyde, | 60 % |
| 2,4 bis-propylamino-6-diméthylamino pyrimidine 3-oxyde, | 55 % |
| 2,4 bis-propylamino-6-pipéridino pyrimidine 3-oxyde, | 57 % |
| 2 amino-4-méthylamino-6-pipéridino pyrimidine 3-oxyde, | 50 % |
| 2-amino-4-propylamino-6-pipéridino pyrimidine 3-oxyde, | 46 % |
| 2 amino-4-hexylamino-6-pipéridino pyrimidine 3-oxyde, | 47 % |
| 2 amino-4-benzylamino-6-pipéridino pyrimidine 3-oxyde, | 45 % |
| 2 amino-4-(2-hydroxyéthylamino)-6-pipéridino pyrimidine 3-oxyde, | 43 % |
| 2-amino-4-propylamino-6-diméthylamino pyrimidine 3-oxyde, | 50 % |
| 2-amino-4-butylamino-6-diméthylamino pyrimidine 3-oxyde, | 51 % |
| 2-amino-4-isopropylamino-6-diméthylamino pyrimidine 3-oxyde, | 46 % |

Ces résultats montrent que les dérivés de pyrimidine substitués en 6 testés présentent des propriétés de stimulateur de la tyrosinase supérieures au Minoxidil.

Exemple 2 : Exemples de compositions contenant un aryl 2,4 dioxo oxazolidine.

Ces compositions sont obtenues par les techniques habituelles couramment utilisées en cosmétique ou en pharmacie.

| Crème dermique : | |
|---|---|
| 2,4 bis-propylamino-6-pipéridino pyrimidine 3-oxyde | 1,000 g |
| Ceteareth 30 | 7,000 g |
| Stéarate de glycéryle | 2,000 g |
| Alcool cétylique | 1,500 g |
| Polydiméthylsiloxane | 1,500 g |
| Huile de paraffine | 15,000 g |

(suite)

| Crème dermique : | |
|---|---|
| Glycérine codex pure | 20,000 g |
| Conservateurs q.s. | |
| Eau déminéralisée q.s.p. | 100,000 g |

| Lotion dermique à pulvériser : | |
|---|---|
| 2,4 bis-méthylamino-6-diméthylamino pyrimidine 3-oxyde | 5,000 g |
| Ethanol | 30,000 g |
| Eau déminéralisée q.s.p. | 100,000 g |

| Lotion pour les cheveux : | |
|---|---|
| 2,4 bis-éthylamino-6-diméthylamino pyrimidine 3-oxyde | 3,000 g |
| Propylène glycol | 30,000 g |
| Alcool éthylique | 40,500 g |
| Eau qsp | 100,000 g |

On applique cette lotion sur le cuir chevelu, une à deux fois par jour, à raison d'1 ml par application.

| Lotion épaissie : | |
|---|---|
| 2,4 bis-propylamino-6-pipéridino pyrimidine 3-oxyde | 5,000 g |
| Kawaïne | 2,000 g |
| Hydroxypropylcellulose (Klucel G de la société Hercules) | 3,500 g |
| Alcool éthylique qsp | 100,000 g |

On applique cette lotion épaissie sur le cuir chevelu, une à deux fois par jour, à raison d'1 ml par application.

| Lotion niosomée : | | |
|---|---|---|
| Chimexane NL® | | 0,475 g |
| Cholestérol | | 0,475 g |
| Stéaroylglutamate monosodique | | 0,050 g |
| 2,4 bis-propylamino-6-pipéridino pyrimidine 3-oxyde | | 0,100 g |
| Conservateurs | qs | |
| Colorants | qs | |
| Parfum | qs | |
| Eau déminéralisée | qsp | 100,000 g |

On applique cette lotion sur le cuir chevelu, une à deux fois par jour, à raison d'1 ml par application.

| Lotion : | |
|---|---|
| 2,4 bis-propylamino-6-diméthylamino pyrimidine 3-oxyde | 5,000 g |
| Monométhyléther de propylèneglycol (Dowanol PM de Dow Chemical) | 20,000 g |
| Hydroxypropylcellulose (Klucel G de la société Hercules) | 3,000 g |
| Alcool éthylique | 40,000 g |

(suite)

| Lotion : | |
|---|---|
| Minoxidil | 2,000 g |
| Eau qsp | 100,000 g |

On applique cette lotion épaissie sur le cuir chevelu, une à deux fois par jour, à raison d'1 ml par application.

## Revendications

1. Utilisation dans une composition cosmétique ou pour la préparation d'une composition pharmaceutique, à titre de principe actif, d'une quantité suffisante d'au moins un dérivé de pyrimidine 3-oxyde, substitué en 6 répondant à la formule générale (I)

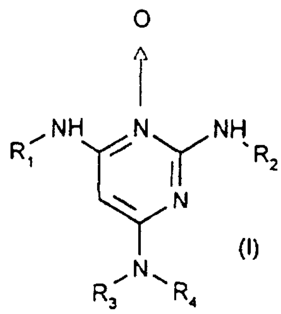

(I)

dans laquelle

$R_1$ et $R_2$ identiques ou différents, représentent un atome d'hydrogène ou un radical alkyle en $C_1$ - $C_{12}$, éventuellement substitué par au moins un radical hydroxyle ou un radical benzyle et en particulier les formes linéaires ou ramifiées éventuellement substituées des radicaux méthyle, éthyle, propyle, butyle, pentyle, hexyle ou encore benzyle.
$R_3$ et $R_4$ identiques ou différents, représentent un radical alkyle en $C_1$ - $C_{12}$, éventuellement substitué par au moins un radical hydroxyle ou un radical benzyle et en particulier les formes linéaires ou ramifiées éventuellement substituées des radicaux méthyle, éthyle, propyle, butyle, pentyle, hexyle ou encore benzyle ou pris ensemble peuvent former avec l'atome d'azote auquel ils sont reliés un hétérocycle ;

étant entendu que lorsque R3 et R4 pris ensemble forment un cycle pipéridino alors au moins l'un des radicaux R1 ou R2 doit être différent d'un atome d'hydrogène.
ce dérivé ou la composition étant destiné à favoriser la pigmentation de la peau et/ou des cheveux.

2. Utilisation dans une composition cosmétique ou pour la préparation d'une composition pharmaceutique, à titre de principe actif, d'une quantité suffisante d'au moins un dérivé de pyrimidine 3-oxyde, substitué en 6 répondant à la formule générale (I)

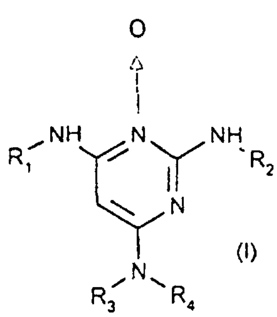

(I)

dans laquelle

$R_1$ et $R_2$. identiques ou différents, représentent un atome d'hydrogène ou un radical alkyle en $C_1$ - $C_{12}$ ;
$R_3$ et $R_4$ identiques ou différents, représentent un radical alkyle en $C_1$ - $C_{12}$, ou pris ensemble peuvent former avec l'atome d'azote auquel ils sont reliés un hétérocycle ;

étant entendu que lorsque R3 et R4 pris ensemble forment un cycle pipéridino alors au moins l'un des radicaux R1 ou R2 doit être différent d'un atome d'hydrogène.
ce dérivé ou la composition étant destiné à stimuler l'activité tyrosinase.

**3.** Utilisation selon l'une quelconque des revendications 1 ou 2, **caractérisée par le fait que** le dérivé de pyrimidine 3-oxyde, substitué en 6 est choisi parmi :

2,4 bis-méthylamino-6-diméthylamino pyrimidine 3-oxyde,
2,4 bis-éthylamino-6-diméthylamino pyrimidine 3-oxyde,
2,4 bis-propylamino-6-diméthylamino pyrimidine 3-oxyde,
2,4 bis-propylamino-6-pipéridino pyrimidine 3-oxyde,
2 amino-4-méthylamino-6-pipéridino pyrimidine 3-oxyde,
2-amino-4-propylamino-6-pipéridino pyrimidine 3-oxyde,
2 amino-4-hexylamino-6-pipéridino pyrimidine 3-oxyde,
2 amino-4-benzylamino-6-pipéridino pyrimidine 3-oxyde,
2 amino-4-(2-hydroxyéthylamino)-6-pipéridino pyrimidine 3-oxyde,
2-amino-4-propylamino-6-diméthylamino pyrimidine 3-oxyde,
2-amino-4-butylamino-6-diméthylamino pyrimidine 3-oxyde,
2-amino-4-isopropylamino-6-diméthylamino pyrimidine 3-oxyde,

**4.** Utilisation selon l'une quelconque des revendications 1 à 3, **caractérisée par le fait que** le dérivé de pyrimidine 3-oxyde, substitué en 6 est choisi parmi :

le 2-amino-4-propylamino-6-diméthylamino pyrimidine 3-oxyde,
le 2 amino-4-méthylamino-6-pipéridino pyrimidine 3-oxyde,
le 2,4 bis-propylamino-6-diméthylamino pyrimidine 3-oxyde,
le 2,4 bis-propylamino-6-pipéridino pyrimidine 3-oxyde,
le 2,4 bis-méthylamino-6-diméthylamino pyrimidine 3-oxyde,
le 2,4 bis-éthylamino-6-diméthylamino pyrimidine 3-oxyde.

**5.** Utilisation dans une composition cosmétique selon l'une quelconque des revendications 1 à 4, **caractérisée par le fait que** le dérivé est utilisé en une quantité comprise entre 0,01% et 20% du poids total de la composition et préférentiellement comprise entre 0,1% et 10% du poids total de la composition.

**6.** Utilisation dans une composition pharmaceutique selon l'une quelconque des revendications 1 à 4, **caractérisée par le fait que** le dérivé est utilisé en une quantité comprise entre 1% et 30% du poids total de la composition et préférentiellement comprise entre 2% et 15% du poids total de la composition.

**7.** Composition cosmétique ou pharmaceutique comprenant au moins un dérivé répondant à la formule générale (I) tel que défini auxrevendications 1 à 6 et un substrat d'au moins une enzyme présentant une activité tyrosinase.

**8.** Composition selon la revendication 7, **caractérisée par le fait que** le substrat utilisable selon l'invention est choisi parmi la tyrosine ou ses dérivés ou la dihydroxy-3,4 phényl $\alpha$-alanine (DOPA).

**9.** Composition selon l'une quelconque des revendications 7 ou 8, **caractérisée par le fait que** le dérivé répondant à la formule générale (I) et le substrat de l'enzyme sont conditionnés séparément.

**10.** Composition selon l'une quelconque des revendications 7 à 9, **caractérisée par le fait qu'**elle est destinée à favoriser la pigmentation de la peau et/ou des cheveux.

**11.** Composition selon l'une quelconque des revendications 7 à 9, **caractérisée par le fait qu'**elle est destinée à stimuler l'activité tyrosinase endogène de la peau et/ou des cheveux.

**12.** Produit comprenant au moins un dérivé répondant à la formule générale (I) et au moins un substrat d'au moins une enzyme présentant une activité tyrosinase comme produit de combinaison pour une utilisation simultanée, séparée ou étalée dans le temps pour favoriser la pigmentation de la peau et/ou des cheveux.

**Patentansprüche**

**1.** Verwendung mindestens eines in 6-Stellung substituierten Pyrimidin-3-oxidderivats der allgemeinen Formel (I) in einer als Wirkstoff ausreichenden Menge in einer kosmetischen Zusammensetzung oder zur Herstellung einer pharmazeutischen Zusammensetzung

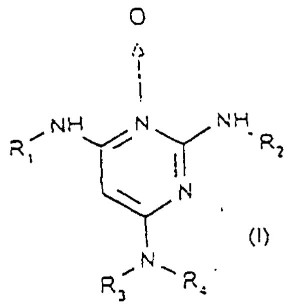

(I)

worin:

die Gruppen $R_1$ und $R_2$, die identisch oder voneinander verschieden sind, Wasserstoff oder eine $C_{1-12}$-Alkylgruppe, die mit mindestens einer Hydrogruppe substituiert ist, oder eine Benzylgruppe bedeuten, insbesondere die geradkettigen oder verzweigten, gegebenenfalls substituierten Formen der Gruppen Methyl, Ethyl, Propyl, Butyl, Pentyl oder Hexyl oder Benzyl; und

die Gruppen $R_3$ und $R_4$, die identisch oder voneinander verschieden sind, eine $C_{1-12}$-Alkylgruppe, die mit mindestens einer Hydroxygruppe substituiert ist, oder eine Benzylgruppe bedeuten, insbesondere die geradkettigen oder verzweigten, gegebenenfalls substituierten Formen der Gruppen Methyl, Ethyl, Propyl, Butyl, Pentyl oder Hexyl oder Benzyl, oder sie bilden gemeinsam mit dem Stickstoffatom, an das sie gebunden sind, einen Heterocyclus;

mit der Maßgabe, daß mindestens eine der Gruppen $R_1$ oder $R_2$ von Wasserstoff verschieden sein muß, wenn $R_3$ und $R_4$ gemeinsam einen Piperidino-Ring bilden,

wobei das Derivat oder die Zusammensetzung dazu vorgesehen sind, die Pigmentierung der Haut und/oder der Haare zu stimulieren.

**2.** Verwendung mindestens eines in 6-Stellung substituierten Pyrimidin-3-oxidderivats der allgemeinen Formel (I) in einer als Wirkstoff ausreichenden Menge in einer kosmetischen Zusammensetzung oder zur Herstellung einer pharmazeutischen Zusammensetzung:

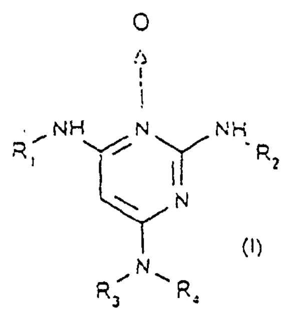

(I)

worin:

die Gruppen $R_1$ und $R_2$, die identisch oder voneinander verschieden sind, Wasserstoff oder eine $C_{1-12}$-Alkylgruppe bedeuten; und
die Gruppen $R_3$ und $R_4$, die identisch oder voneinander verschieden sind, eine $C_{1-12}$-Alkylgruppe bedeuten oder gemeinsam mit dem Stickstoffatom, an das sie gebunden sind, einen Heterocyclus bilden; mit der Maßgabe, daß mindestens eine der Gruppen $R_1$ oder $R_2$ von Wasserstoff verschieden sein muß, wenn $R_3$ und $R_4$ gemeinsam einen Piperidino-Ring bilden,

wobei das Derivat oder die Zusammensetzung dazu vorgesehen sind, die Tyrosinaseaktivität zu stimulieren.

**3.** Verwendung nach einem der Ansprüche 1 oder 2, **dadurch gekennzeichnet, daß** das in 6-Stellung substituierte Pyrimidin-3-oxidderivat ausgewählt ist unter:

2,4-Bis-methylamino-6-dimethylamino-pyrimidin-3-oxid,
2,4-Bis-ethylamino-6-dimethylamino-pyrimidin-3-oxid,
2,4-Bis-propylamino-6-dimethylamino-pyrimidin-3-oxid,
2,4-Bis-propylamino-6-piperidino-pyrimidin-3-oxid,
2-Amino-4-methylamino-6-piperidino-pyrimidin-3-oxid,
2-Amino-4-propylamino-6-piperidino-pyrimidin-3-oxid,
2-Amino-4-hexylamino-6-piperidino-pyrimidin-3-oxid,
2-Amino-4-benzylamino-6-piperidino-pyrimidin-3-oxid,
2-Amino-4-(2-hydroxyethylamino)-6-piperidino-pyrimidin-3-oxid,
2-Amino-4-propylamino-6-dimethylamino-pyrimidin-3-oxid,
2-Amino-4-butylamino-6-dimethylamino-pyrimidin-3-oxid, und
2-Amino-4-isopropylamino-6-dimethylamino-pyrimidin-3-oxid,

**4.** Verwendung nach einem der Ansprüche 1 bis 3, **dadurch gekennzeichnet, daß** das in 6-Stellung substituierte Pyrimidin-3-oxidderivat ausgewählt ist unter:

2-Amino-4-propylamino-6-dimethylamino-pyrimidin-3-oxid,
2-Amino-4-methylamino-6-piperidino-pyrimidin-3-oxid,
2,4-Bis-propylamino-6-dimethylamino-pyrimidin-3-oxid,
2,4-Bis-propylamino-6-piperidino-pyrimidin-3-oxid,
2,4-Bis-methylamino-6-dimethylamino-pyrimidin-3-oxid, und
2,4-Bis-ethylamino-6-dimethylamino-pyrimidin-3-oxid.

**5.** Verwendung nach einem der Ansprüche 1 bis 4 in einer kosmetischen Zusammensetzung, **dadurch gekennzeichnet, daß** das Derivat in einer Menge im Bereich von 0,01 bis 20 % des Gesamtgewichts der Zusammensetzung und vorzugsweise in einer Menge von 0,1 bis 10 % des Gesamtgewichts der Zusammensetzung verwendet wird.

**6.** Verwendung nach einem der Ansprüche 1 bis 4 in einer pharmazeutischen Zusammensetzung, **dadurch gekennzeichnet, daß** das Derivat in einer Menge im Bereich von 1 bis 30 % des Gesamtgewichts der Zusammensetzung und vorzugsweise in einer Menge von 2 bis 15 % des Gesamtgewichts der Zusammensetzung verwendet wird.

**7.** Kosmetische oder pharmazeutische Zusammensetzung, die mindestens ein Derivat der allgemeinen Formel (I) nach einem der Ansprüche 1 bis 6 und ein Substrat mindestens eines Enzyms, das eine Tyrosinase-Aktivität besitzt, enthält.

**8.** Zusammensetzung nach Anspruch 7, **dadurch gekennzeichnet, daß** das erfindungsgemäß verwendbare Substrat unter Tyrosin oder seinen Derivaten oder $\alpha$-(3,4-Dihydroxyphenyl)-alanin (DOPA) ausgewählt ist.

**9.** Zusammensetzung nach einem der Ansprüche 7 oder 8, **dadurch gekennzeichnet, daß** das Derivat der allgemeinen Formel (I) und das Substrat des Enzyms getrennt voneinander konfektioniert sind.

**10.** Zusammensetzung nach einem der Ansprüche 7 bis 9, **dadurch gekennzeichnet, daß** sie dazu vorgesehen ist, die Pigmentierung der Haut und/oder der Haare zu stimulieren.

**11.** Zusammensetzung nach einem der Ansprüche 7 bis 9, **dadurch gekennzeichnet, daß** sie dazu vorgesehen ist,

die endogene Tyrosinase-Aktivität der Haut und/oder der Haare zu stimulieren.

**12.** Produkt, das mindestens ein Derivat der allgemeinen Formel (I) und mindestens ein Substrat mindestens eines Enzyms enthält, das Tyrosinase-Aktivität besitzt, als Kombinationsprodukt für eine gemeinsame, getrennte oder zeitlich aufeinanderfolgende Verwendung, um die Figmentierung der Haut und/oder der Haare zu stimulieren.

**Claims**

**1.** Use in a cosmetic composition or for the preparation of a pharmaceutical composition, as active principle, of a sufficient amount of at least one pyrimidine 3-oxide derivative, substituted in position 6, corresponding to the general formula (I)

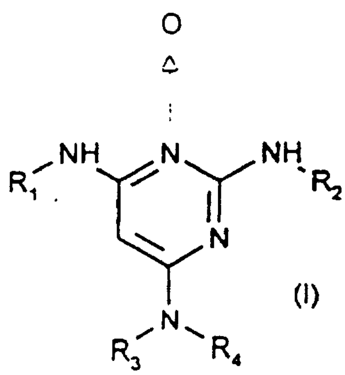

(I)

in which

$R_1$ and $R_2$, which may be identical or different, represent a hydrogen atom or a $C_1$-$C_{12}$ alkyl radical, optionally substituted with at least one hydroxyl radical or a benzyl radical and in particular the optionally substituted, linear or branched forms of the methyl, ethyl, propyl, butyl, pentyl, hexyl or else benzyl radicals;

$R_3$ and $R_4$, which may be identical or different, represent a $C_1$-$C_{12}$ alkyl radical, optionally substituted with at least one hydroxyl radical or a benzyl radical and in particular the optionally substituted, linear or branched forms of the methyl, ethyl, propyl, butyl, pentyl, hexyl or else benzyl radicals or, taken together, may form a heterocycle with the nitrogen atom to which they are attached;

it being understood that when R3 and R4, taken together, form a piperidino ring, then at least one of the radicals R1 or R2 must be other than a hydrogen atom;

this derivative or the composition being intended to promote pigmentation of the skin and/or the hair.

**2.** Use in a cosmetic composition or for the preparation of a pharmaceutical composition, as active principle, of a sufficient amount of at least one pyrimidine 3-oxide derivative, substituted in position 6, corresponding to the general formula (I)

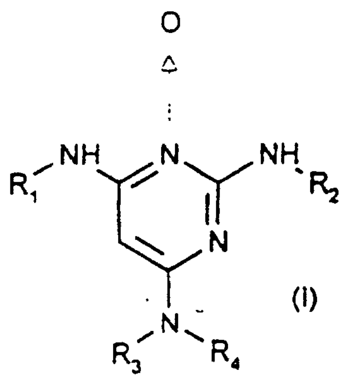

(I)

in which

$R_1$ and $R_2$, which may be identical or different, represent a hydrogen atom or a $C_1$-$C_{12}$ alkyl radical;

$R_3$ and $R_4$, which may be identical or different, represent a $C_1$-$C_{12}$ alkyl radical or, taken together, may form a heterocycle with the nitrogen atom to which they are attached;
it being understood that when R3 and R4, taken together, form a piperidino ring, then at least one of the radicals R1 or R2 must be other than a hydrogen atom,
this derivative or the composition being intended to stimulate tyrosinase activity.

**3.** Use according to either of Claims 1 and 2, **characterized in that** the pyrimidine 3-oxide derivative substituted in position 6 is chosen from:

2,4-bis-methylamino-6-dimethylaminopyrimidine 3-oxide,
2,4-bis-ethylamino-6-dimethylaminopyrimidine 3-oxide,
2,4-bis-propylamino-6-dimethylaminopyrimidine 3-oxide,
2,4-bis-propylamino-6-piperidinopyrimidine 3-oxide,
2-amino-4-methylamino-6-piperidinopyrimidine 3-oxide,
2-amino-4-propylamino-6-piperidinopyrimidine 3-oxide,
2-amino-4-hexylamino-6-piperidinopyrimidine 3-oxide,
2-amino-4-benzylamino-6-piperidinopyrimidine 3-oxide,
2-amino-4-(2-hydroxyethylamino)-6-piperidinopyrimidine 3-oxide,
2-amino-4-propylamino-6-dimethylaminopyrimidine 3-oxide,
2-amino-4-butylamino-6-dimethylaminopyrimidine 3-oxide,
2-amino-4-isopropylamino-6-dimethylaminopyrimidine 3-oxide.

**4.** Use according to any one of Claims 1 to 3, **characterized in that** the pyrimidine 3-oxide derivative substituted in position 6 is chosen from:

2-amino-4-propylamino-6-dimethylaminopyrimidine 3-oxide,
2-amino-4-methylamino-6-piperidinopyrimidine 3-oxide,
2,4-bis-propylamino-6-dimethylaminopyrimidine 3-oxide,
2,4-bis-propylamino-6-piperidinopyrimidine 3-oxide,
2,4-bis-methylamino-6-dimethylaminopyrimidine 3-oxide,
2,4-bis-ethylamino-6-dimethylaminopyrimidine 3-oxide.

**5.** Use in a cosmetic composition according to any one of Claims 1 to 4, **characterized in that** the derivative is used in an amount of between 0.01% and 20% of the total weight of the composition and preferably between 0.1% and 10% of the total weight of the composition.

**6.** Use in a pharmaceutical composition according to any one of Claims 1 to 4,**characterized in that** the derivative is used in an amount of between 1% and 30% of the total weight of the composition and preferably between 2% and 15% of the total weight of the composition.

**7.** Cosmetic or pharmaceutical composition comprising at least one derivative corresponding to the general formula (I) as defined in Claims 1 to 6 and a substrate of at least one enzyme having a tyrosinase activity.

**8.** Composition according to Claim 7, **characterized in that** the substrate which may be used according to the invention is chosen from tyrosine or its derivatives, or 3,4-dihydroxy-$\alpha$-phenylalanine (DOPA).

**9.** Composition according to either of Claims 7 and 8, **characterized in that** the derivative corresponding to the general formula (I) and the substrate of the enzyme are packaged separately.

**10.** Composition according to any one of Claims 7 to 9, **characterized in that** it is intended to promote pigmentation of the skin and/or the hair.

**11.** Composition according to any one of Claims 7 to 9, **characterized in that** it is intended to stimulate the endogenous tyrosinase activity of the skin and/or the hair.

**12.** Product comprising at least one derivative corresponding to the general formula (I) and at least one substrate of at least one enzyme having a tyrosinase activity, as a combination product for a simultaneous or separate use or for use spread out over time, in order to promote pigmentation of the skin and/or the hair.